# EUROPÄISCHE PATENTANMELDUNG

(11) **EP 1 180 357 A2**
(43) Veröffentlichungstag der Anmeldung: **20.02.2002**
(21) Anmeldenummer: 01118398.5
(22) Anmeldetag: 28.07.2001
(51) Int. Cl.: A61K 7/06

(54) **Verfahren zum Behandeln von menschlichen Haaren**

(30) Priorität: 18.08.2000 DE 10040513
(71) Anmelder: GOLDWELL GmbH, 64280 Darmstadt (DE)
(72) Erfinder: Fath, Bettina, 69469 Weinheim (DE); Dubowoj, Polina, Aree PLace Condominium Room 801, Klongton, Klongtoey, Bangkok 10110 (TH)

(57) **Zusammenfassung**

Die vorliegende Erfindung betrifft ein Verfahren zur Behandlung von menschlichem Haar, wobei eine Zusammensetzung aufgebracht wird, die, mindestens ein pulverförmiges Polyamid, von dem mindestens 80% einen Teilchendurchmesser zwischen 1 und 20 Mikron aufweisen, und mindestens ein haarkonditionierendes Mittel enthält.
Das Mittel wird aus dem Haar ausgespült und verleiht diesem Glanz, Volumen und einen vollen und lockeren Griff, festen Halt und eine verbesserte Naß- und Trockenkämmbarkeit.

## Beschreibung

Die vorliegende Erfindung betrifft ein Verfahren zum Behandeln von menschlichem Haar, das diesem verbesserte Eigenschaften, insbesondere Naß- und Trockenkämmbarkeit, erhöhten Glanz, einen vollen und gleichzeitig lockeren Griff sowie guten Halt verleiht.

Mittel zum Konditionieren von menschlichen Haaren sind seit langem bekannt.
Sie enthalten in der Regel quaternäre Ammoniumverbindungen, die mindestens eine langkettige Alkyl- oder Alkenylgruppe aufweisen, und gegebenenfalls auch Polymere.
Solche Mittel werden üblicherweise als wäßrige Dispersionen bzw. Emulsionen, Mikroemulsionen, Gele oder auch in Aerosolform konfektioniert und als Haarspülungen, Kuren, etc. eingesetzt.
Eine Übersicht über die bekannten Haarnachbehandlungsmittel und ihre Zusammensetzung findet sich in der Monographie von K. Schrader, Grundlagen und Rezepturen der Kosmetika, 2. Aufl. (1989), S. 722 bis 781, insbesondere S. 728 bis 737.
Diese bekannten Zusammensetzungen sind aber noch verbesserungsfähig.

Es wurde nunmehr gefunden, daß durch die Anwendung eines Haarbehandlunsmittels dem Haar verbesserte Eigenschaften, insbesondere ein deutlich erhöhter Volumeneffekt, leichtere Naß- und Trockenkämmbarkeit sowie einen dezenter Glanz, ein voller lockerer Griff und guter Halt verliehen werden, wenn dieses Mittel auf wäßriger Basis mindestens ein pulverförmiges Polyamid mit einem Teilchendurchmesser von mindestens 80% im Bereich von 1 bis 20 Mikron, und mindestens einen haarkonditionierenden Wirkstoff enthält.
Dieses Mittel wird, vorzugsweise nach einer Haarwäsche, gegebenenfalls im Anschluß an eine zuvor durchgeführte Haarfärbung oder Dauerwellung, auf das Haar aufgebracht, vorzugsweise in dieses einmassiert und nach der Einwirkung nach etwa 1 bis 25 Minuten wieder ausgespült.

Es handelt sich also ausschließlich um sogenannte "Rinse off"-Produkte, die nach einer kurzen Einwirkungszeit wieder aus dem Haar ausgespült werden, im Gegensatz zu sogenannten "Leave on"-Produkten, die nach dem Aufbringen auf dem Haar verbleiben.

Geeignete pulverförmige Polyamide, die in den erfindungsgemäß zur Haarbehandlung verwendeten Mitteln vorzugsweise in einer Menge von etwa 0,1 bis etwa 10, insbesondere etwa 0,25 bis etwa 7,5, vor allem etwa 0,5 bis 5 Gew.-%, bezogen auf die Gesamtzusammensetzung des Mittels, eingesetzt werden, sind an sich bekannt, beispielsweise als "Polyamide 6" oder "12" unter der Bezeichnung "Nylon". 80% der Teilchen weisen dabei einen Teilchendurchmesser im Bereich von 1 bis 20, vorzugsweise etwa 2,5 bis 17, insbesondere etwa 5 bis 15 Mikron auf.

Weitere Handelsprodukte sind "Orgasol^{R}1002", "2002" und "4000".

Geeignete haarkonditionierende Wirkstoffe, deren Menge in den erfindungsgemäß verwendeten Mitteln üblicherweise bei etwa 0,25 bis etwa 15, insbesondere 0,5 bis etwa 10, vorzugsweise etwa 1 bis 7,5 Gew.-%, bezogen auf die Gesamtmenge des Mittels liegt, sind insbesondere kationische Tenside, vor allem quaternäre Ammoniumverbindungen.

Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Dimethyldistearylammoniumchlorid, Stearyltrimethylammoniumchlorid, Dimethylstearylbenzylammoniumchlorid, Benzyltetradecylodimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc.

Gut geeignet sind auch die in der EP-A 472 107 geoffenbarten quaternären Ammoniumsalze.

Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.
Geeignet sind auch langkettige Amine, z.B. Stearamidopropyldimethylamin.

Der Anteil dieser obengenannten Verbindungen liegt vorzugsweise bei etwa 0,5 bis 10, insbesondere etwa 1 bis 7,5 Gew.-%, berechnet auf die Gesamtzusammensetzung.

Besonders geeignete langkettige quaternäre Ammoniumverbindungen sind Esterquats der allgemeinen Formel (I) 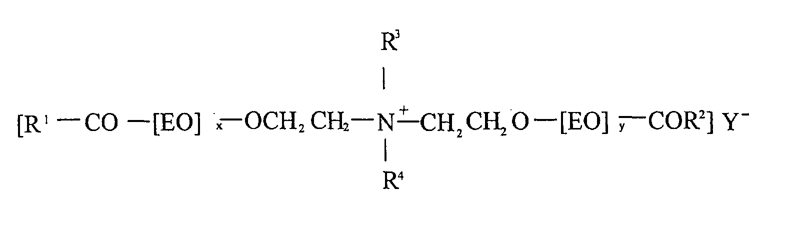 in der R¹ und R² für eine gegebenenfalls hydroxysübstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Ein besonders bevorzugtes Esterquat ist im Rahmen der Erfindung ein solches, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{A}", "Dehyquart^{R}F30" und "Tetranyl^{R}" im Handel.

Der Einsatz dieser Verbindungen in Haarpflegemitteln ist ebenfalls bereits bekannt und beispielsweise in der WO-A 93/10748, der WO-A 92/06899 und der WO-A 94/16677 beschrieben, wo sich jedoch keinerlei Hinweise auf die erfindungsgemäße Kombination und deren vorteilhafte Eigenschaften finden.

Ebenso geeignet sind "Amidoquats" der allgemeinen Formel 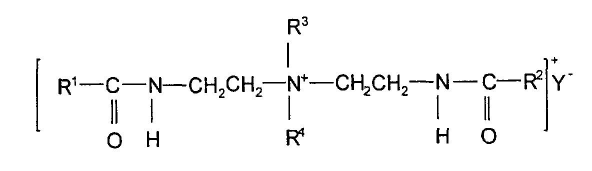 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]ₓ-H, sowie x für 0 bis 5, Y für ein Anion stehen.

Bevorzugte Reste R¹ und R² sind C₁₂-C₁₈-Alkyl- und Oleylreste, der Rest R³ eine Methylgruppe und der Rest R⁴ eine Gruppe -CH₂-CH₂-O[EO]ₓ-H, worin x 0 bis 3 ist; Y⁻ ist vorzugsweise ein Methosulfat-, Ethylsulfat-, Chlorid oder Phosphatanion.

Diese Verbindungen sind an sich bekannt und beispielsweise unter den Markenbezeichnungen "INCROQUAT® HO" oder "OCS" im Handel.

Weitere geeignete haarkonditionierende Wirkstoffe sind synthetische oder natürliche haarkonditionierende Polymere, vorzugsweise in einer Menge von 0,1 bis 10, insbesondere 0,25 bis 5 Gew.-% der Gesamtzusammensetzung.

Besonders bevorzugt sind hierbei die unter der CTFA-Bezeichnung "Polyquaternium" bekannten kationischen (Co-)Polymeren, jedoch können auch nichtionische, anionische und/oder amphotere Polymere, beispielsweise solche vom Typ "Amphomer^{R}", alleine oder im Gemisch verwendet werden.

Weitere haarkonditionierende Wirkstoffe sind lipophile, d.h. fett- und ölhaltige Substanzen, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.
Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäß verwendeten Zusammensetzungen vorzugsweise in einer Gesamtmenge von etwa 0,5 bis etwa 15, insbesondere etwa 1 bis 10, vor allem etwa 1,5 bis 7,5 Gew.-%, berechnet auf die Gesamtzusammensetzung, enthalten.

Weitere geeignete haarkonditionierende Zusatzstoffe sind Ceramide der allgemeinen Formel 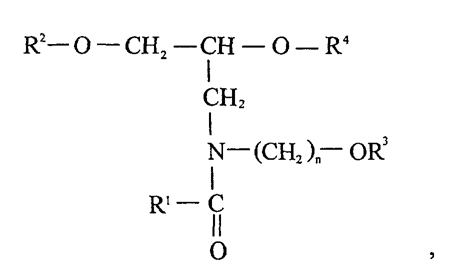 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyl- oder Isopropylgruppe steht, R⁴ Wasserstoff, eine Hydroxymethyl-,Hydroxyethyl-, Dihydroxyethyl- oder Dihydroxypropylgruppe, und n eine ganze Zahl von 1 bis 6 bedeuten, insbesondere der Art, wie es aus der EP 227 994 A1 und der WO-A 96/37462 bekannt ist, jedoch sind auch andere Ceramide, beispielsweise die aus der WO-A 97/15724 oder der EP 647 617 B1 bekannten Ceramide, geeignet.

Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄-C₁₈-Alkylrest, insbesondere eine C₁₆H₃₃-Alkylgruppe, R³ einen Methylrest, R⁴ eine 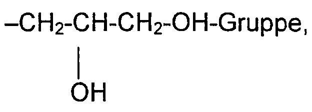 und n 3 darstellen, oder eine Verbindung, wo R¹ für einen C₁₅-C₃₁-Alkylrest, R² für einen C₁₆H₃₃-Alkylrest, R³ und R⁴ für je ein Wasserstoffatom und n für 2 stehen.

Deren Menge in den erfindungsgemäß eigesetzten Haarnachspülmitteln liegt zweckmäßigerweise bei etwa 0,01 bis 10, vorzugsweise etwa 0,05 bis 7,5, insbesondere etwa 0,1 bis 5 Gew.-%, berechnet auf die Gesamtzusammensetzung.

Weitere geeignete haarkonditionierende Wirkstoffe sind wasserunlösliche Vitamine und deren Derivate, beispielsweise Vitamin E und dessen Ester wie Tocopherolacetat, -propionat, -palmitat, etc.

Weitere Zusatzstoffe, deren Art und Menge natürlich von der Applikationsform des Mittels abhängig sind, sind Fette, Fettalkohole, Emulgatoren, pH-Regulatoren, Lösungs- und Verdünnungsmittel, Lösungsvermittler, Konservierungsmittel, Parfums, etc.

Als Tenside sind insbesondere nichtionische Tenside geeignet.
Besonders bevorzugte nichtionische Tenside sind die bekannten C₈-C₁₈-Alkylpolyglucoside, insbesondere mit einem Polykondensationsgrad von 1,2 bis 3, vor allem solche der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z. einen Saccharidrest mit 5 bis 6 Kohlenstoffatomen, n eine Zahl von 0 bis 10 und x eine Zahl zwischen 1 und 2,5 bedeuten, in einer Menge von 1 bis 10, insbesondere 2,5 bis 7,5 Gew.-% der Gesamtzusammensetzung.

Andere geeignete nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.
Daneben sind natürlich auch anionische und/oder amphotere bzw. zwitterionische Tenside geeignet.

Die erfindungsgemäß verwendeten auszuspülenden haarkonditionierenden Mittel liegen vorzugsweise als wäßrige oder wäßriglalkoholische Lösung, wäßrige Emulsion, Mikroemulsion, Dispersion oder opakes oder transparentes Gel vor und können auch als Aerosole konfektioniert werden. Solche Zusammensetzungen und ihre Herstellung sind dem Fachmann grundsätzlich bekannt und bedürfen daher keiner näheren Erläuterung.

Der pH-Wert der erfindungsgemäß verwendeten Haarnachbehandlungsmittel ist nicht kritisch; er kann vorzugsweise bei 3 bis etwa 8, insbesondere zwischen 4 und 6,5, liegen.

Ebenso liegt die Viskosität im allgemein Bereich und ist selbstverständlich abhängig von der Darreichungsform des Mittels.

Die folgenden Beispiele beschreiben die Erfindung im Detail:

### Beispiel 1

Es wurde eine Haarkur folgender Zusammensetzung hergestellt:

| | |
|---|---|
| Cetylstearylalkohol | 6,5 (Gew.-%) |
| Isopropylmyristat | 1,0 |
| Steartrimoniumchlorid | 0,8 |
| Behentrimoniumchlorid | 1,5 |
| Mandelöl | 0,1 |
| Parfum | 0,3 |
| Lecithin | 0,6 |
| Konservierungsmittel | 0,3 |
| Nylon-12 (mittl. Teilchendurchmesser: 90% zwischen 3,5 und 12,5 µm) | 0,5 |
| Citronensäure | 0,1 |
| Wasser | ad 100,0 |
| pH-Wert: | ∼4,5 |
| Viskosität bei 20°C: (Brookfield, Spindel Nr. 4) | ∼30 000 mPa.s |

An strapazierten Haarsträhnen wurde folgender Blindversuch durchgeführt:

In 5 Strähnen wurde nach der Haarwäsche mit einem handelsüblichen Shampoo in das noch nasse Haar eine Zusammensetzung 1 entsprechend dem obigen Beispiel einmassiert.

Weitere 5 Strähnen wurden mit einer mit der Zusammensetzung 1 identischen Zusammensetzung 1A, die jedoch keine Polyamidteilchen enthielt, in gleicher Weise behandelt.

Nach fünfminütiger Einwirkung wurde mit Wasser ausgespült und das Haar getrocknet.

Die Haarsträhnen wurden vor und nach dem Trocknen von jeweils 2 Friseuren nach der Präferenzmethode beurteilt.

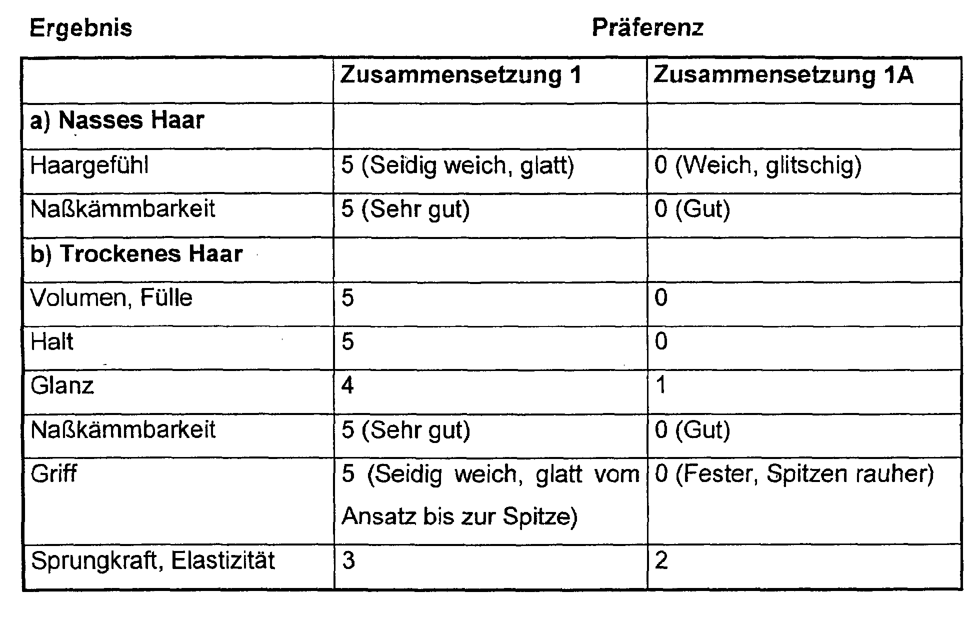

Dieses Ergebnis zeigt die überraschende Überlegenheit des erfindungsgemäßen Verfahrens.

### Beispiel 2

| **"Rinse off"-Kur** | |
|---|---|
| Cetrimoniumchlorid | 1,5 (Gew.-%) |
| Cetylstearylalkohol | 7,0 |
| Quaternium-18 | 2,5 |
| Lanolin | 1,0 |
| Glycerin | 5,0 |
| Citronensäure | 0,1 |
| Parfum | 0,4 |
| Konservierungsmittel | 0,3 |
| Polyamidpulver (Orgasol^{R}2002, durchschnittl. Teilchendurchmesser 5 bis 15µm) | 0,6 |
| Wasser | ad 100,0 |
| pH-Wert: | ∼4,5 |
| Viskosität bei 20°C: (Brookfield, Spindel Nr. 4) | ∼35 000 mPa.s |

Mit diesem Produkt wurde nach zehnminütiger Einwirkung und anschließendem Ausspülen und Trocknen eine ähnliche Verbesserung der Haareigenschaften wie mit demjenigen nach Beispiel 1 erzielt.

### Beispiel 3

| **"Rinse off"-Kur** | |
|---|---|
| Di-C₂₂-C₂₅-alkyldimethylammoniumchlorid | 2,0 (Gew.-%) |
| Steartrimoniumchlorid | 2,3 |
| Cetylstearylalkohol | 4,0 |
| Ceteareth-20 | 3,5 |
| Behensäure | 0,4 |
| Nylon-12 (mittl. Teilchendurchmesser: 90% 3,5 bis 12,5µm) | 0,7 |
| Paraffinöl | 2,0 |
| Weizenproteinhydrolysat (40%) | 1,6 |
| Parfum | 0,4 |
| Glycerin | 1,0 |
| Konservierungsmittel | 0,3 |
| Wasser | ad 100,0 |
| pH-Wert: | ∼4,0 |
| Viskosität bei 20°C: (Brookfield, Spindel Nr. 4) | ∼ 30 000 mPa.s |

Die Anwendung dieses Produktes ergab nach fünfminütiger Einwirkung auf dem Haar und anschließendem Ausspülen und Trocknen ähnlich gute Eigenschaften wie dasjenige nach Beispiel 1.

### Beispiel 4

Eine Haarspülung der folgenden Zusammensetzung

| | |
|---|---|
| Tocopherylacetat | 0,1 (Gew.-%) |
| Cetylstearylalkohol | 3,0 |
| Polyamidpulver (mittl. Teilchengröße: | 0,5 |
| 85% zwischen 4 und 12µm) | |
| Steartrimoniumchlorid | 0,8 |
| Behenyltrimoniumchlorid | 1,2 |
| Isopropylmyristat | 1,0 |
| Paraffinöl | 1,0 |
| Hydroxypropyl Guar Hydroxypropyltrimoniumchlorid | 0,4 |
| Parfum | 0,5 |
| Konservierungsmittel | 0,3 |
| Citronensäure | 0,1 |
| Wasser | ad 100,0 |
| pH-Wert: | ∼3,5 |
| Viskosität bei 20°C: (Brookfield, Spindel Nr. 4) | ∼10 000 mPa.s |

ergab nach dem Einmassieren in frisch gewaschenes Haar eine exzellente haarpflegende Wirksamkeit.

## Patentansprüche

1. Verfahren zum Behandeln von menschlichem Haar, **dadurch gekennzeichnet, daß** auf dieses eine Zusammensetzung auf wäßriger Basis, enthaltend mindestens ein pulverförmiges Polyamid mit einem Teilchendurchmesser von mindestens 80% im Bereich von 1 bis 20 Mikron, und mindestens einen haarkonditionierenden Wirkstoff, aufgebracht und nach etwa 1 bis 25 Minuten wieder ausgespült wird.

2. Verfahren nach Anspruch 1, **dadurch gekennzeichnet, daß** die Zusammensetzung etwa 0,1 bis etwa 10 Gew.-% des pulverförmigen Polyamids enthält.

3. Verfahren nach Anspruch 1 oder 2, **dadurch gekennzeichnet, daß** die Zusammensetzung als haarkonditionierenden Wirkstoff mindestens etwa 0,25 bis 15 Gew.-% eines kationischen Tensids enthält.

4. Verfahren nach einem oder mehreren der Ansprüche 1 bis 3, **dadurch gekennzeichnet, daß** die Zusammensetzung als haarkonditionierenden Wirkstoff etwa 0,25 bis 15 Gew.-% eines lipophilen Stoffs enthält.

5. Verwendung einer wäßrigen Spülzusammensetzung, enthaltend ein pulverförmiges Polyamid mit einem Teilchendurchmesser von mindestens 80% im Bereich von 1 bis 15 Mikron, und mindestens einen haarkonditionierenden Wirkstoff, zur Verbesserung der Eigenschaften von menschlichem Haar.
